(19) Europäisches Patentamt
European Patent Office
Office européen des brevets

(11) EP 3 348 554 B1

(12) **EUROPEAN PATENT SPECIFICATION**

(45) Date of publication and mention of the grant of the patent:
**06.05.2020 Bulletin 2020/19**

(21) Application number: **16844205.1**

(22) Date of filing: **26.08.2016**

(51) Int Cl.:
***C07D 401/14*** (2006.01) ***C07D 401/04*** (2006.01)
***C07D 471/04*** (2006.01)

(86) International application number:
**PCT/JP2016/075025**

(87) International publication number:
**WO 2017/043342 (16.03.2017 Gazette 2017/11)**

# (54) METHOD FOR PRODUCING TRIAZOLE COMPOUND

VERFAHREN ZUR HERSTELLUNG EINER TRIAZOLVERBINDUNG

PROCÉDÉ DE PRODUCTION D'UN COMPOSÉ DE TRIAZOLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2015 JP 2015176948**

(43) Date of publication of application:
**18.07.2018 Bulletin 2018/29**

(73) Proprietor: **Sumitomo Chemical Company, Limited**
**Tokyo 104-8260 (JP)**

(72) Inventors:
- **TANI, Kazuyasu**
**Takarazuka-shi**
**Hyogo 665-8555 (JP)**
- **TANIMOTO, Masaya**
**Osaka-shi**
**Osaka 554-8558 (JP)**
- **UJITA, Satoru**
**Takarazuka-shi**
**Hyogo 665-8555 (JP)**

(74) Representative: **Vossius & Partner Patentanwälte Rechtsanwälte mbB**
**Siebertstrasse 3**
**81675 München (DE)**

(56) References cited:
**WO-A1-2013/018928**
**WO-A1-2014/119699**
**WO-A1-2015/133603**
**WO-A2-2007/130780**
**WO-A2-2012/118563**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field

The present invention relates to a method for producing a triazole compound etc.

Background Art

WO2013/018928 describes compounds having control efficacies against pests.

Summary of Invention

The present invention provides a production intermediate of a compound represented by formula (6):

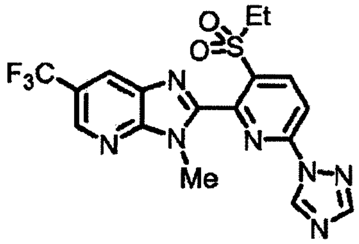

(6)

(hereinafter referred to as compound (6)) having excellent control efficacies against pests and a method for producing the compound (6).

The compound (6) has excellent control efficacies against pests, and a compound represented by formula (5):

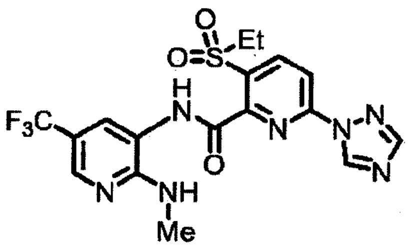

(5)

(hereinafter referred to as compound (5)) is useful as a production intermediate in producing the compound (6) and can be produced by the following method according to the present invention.

Namely, the compound (5) can be produced by

Step (A): adding a compound represented by formula (4)

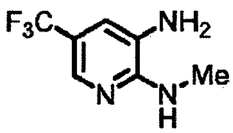

(4)

(hereinafter referred to as compound (4)) and phosphorus oxychloride simultaneously and separately to a compound represented by formula (3)

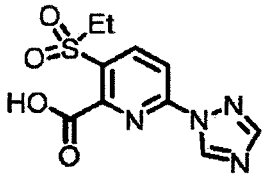

(3)

(hereinafter referred to as compound (3)) to produce the compound (5).

The compound (6) can be produced by

Step (A); and

Step (B) : subjecting the compound (5) to intramolecular condensation in the presence of an acid to produce the compound (6).

Also, the compound (3) can be produced by
Step (C): reacting a compound represented by formula (1)

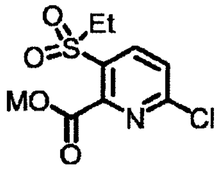

(1)

wherein M represents a sodium atom or a potassium atom (hereinafter referred to as compound (1)) with 1H-1,2,4-triazole, namely a compound represented by formula (2)

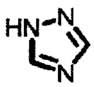

(2)

in the presence of an inorganic base to obtain a reaction mixture, and then mixing the reaction mixture with an acid.

Further, the present invention provides the compound (3) as a production intermediate of the compound (6).

Brief Description of Drawings

Figure 1 shows powder X-ray diffraction of the compound (6) in Example 7.

Description of Embodiments

First, Step (A) is described.

In Step (A), the compound (4) and phosphorus oxychloride are simultaneously and separately added to the compound (3) to produce the compound (5).

In the step, "simultaneous and separate addition of the compound (4) and phosphorus oxychloride to the compound (3)" means that addition of the compound (4) and phosphorus oxychloride is carried out with adjusting the conditions such as addition rate (for example drop rate) so that the molar ratio of the compound (4) and phosphorus oxychloride can be maintained within the range of about 1:1, specifically 1:0.5 to 1:2, and preferably 1:0.8 to 1:1.3 in the reaction system (namely a composition obtained by dropping the compound (4) and phosphorus oxychloride to the compound (3)) of Step (A).

"Simultaneous and separate addition of the compound (4) and phosphorus oxychloride to the compound (3)" can be carried out by, for example, adding the compound (4) and phosphorus oxychloride from separate addition ports to the compound (3) in a container.

The compound (3) is usually mixed with a solvent and used as a liquid composition.

The solvent is an inert solvent, and examples of the solvent include ethers such as 1,4-dioxane, diethyl ether, tetrahydrofuran (THF), tert-butyl methyl ether, cyclohexyl methyl ether, ethylene glycol dimethyl ether, and diethylene glycol dimethyl ether; halogenated hydrocarbons such as dichloromethane, chloroform, carbon tetrachloride, 1,2-dichloroethane, and chlorobenzene; aromatic hydrocarbons such as toluene, benzene, xylene, and ethylbenzene; nitriles such

as acetonitrile; aprotic polar solvents such as N,N-dimethylformamide (DMF), N,N-dimethylacetamide, N-methyl-2-pyrrolidone (NMP), 1,3-dimethyl-2-imidazolidinone, sulfolane, and dimethyl sulfoxide (DMSO); nitrogen-containing aromatic compounds such as pyridine and quinoline; and mixtures thereof.

When the compound (4) is added to the compound (3), the compound (4) is usually mixed with a solvent and used as a solution. Examples of the solvent are the same as described above.

When phosphorus oxychloride is used, it may be diluted with a solvent. The solvent is an inert solvent, and examples of the solvent include ethers such as 1,4-dioxane, diethyl ether, THF, tert-butyl methyl ether, cyclohexyl methyl ether, ethylene glycol dimethyl ether, and diethylene glycol dimethyl ether; halogenated hydrocarbons such as dichloromethane, chloroform, carbon tetrachloride, 1,2-dichloroethane, and chlorobenzene; aromatic hydrocarbons such as toluene, benzene, xylene, and ethylbenzene; nitriles such as acetonitrile ; and mixtures thereof.

The reaction of Step (A) is preferably carried out with the addition of a base. The base is usually preliminarily added to a solution of the compound (4).

Examples of the base include tertiary amines such as triethylamine and N,N-diisopropylethylamine; and nitrogen-containing aromatic compounds such as pyridine and 4-dimethylaminopyridine.

In the reaction, 0.5 to 2 moles, preferably 0.8 to 1.5 moles of the compound (4) are usually used per one mole of the compound (3).

In the reaction, 0.5 to 2 moles, preferably 0.8 to 1.5 moles of phosphorus oxychloride are usually used per one mole of the compound (3).

When a base is used in the reaction, 0.5 to 5 moles, preferably 0.8 to 3 moles of the base are usually used per one mole of the compound (3).

The reaction temperature is usually within the range of -10 to 80°C, preferably 0 to 60°C.

The reaction time is usually within the range of 0.1 to 24 hours, preferably 1 to 20 hours.

When the reaction is completed, the reaction mixture is subjected to work-up, for example, mixed with water, then extracted with organic solvent(s), and the obtained organic layer is dried or concentrated to isolate the compound (5). The isolated compound (5) may be further purified by chromatography, recrystallization, or the like.

The reaction mixture comprising the compound (5) may be directly subjected to the next reaction to produce the compound (6).

Next, Step (B) is described.

The reaction of Step (B) is usually carried out in a solvent.

Examples of the solvent include alcohols such as 2-butanol, ethylene glycol (ethane-1,2-diol), propylene glycol (1,2-propanediol), dipropylene glycol (a mixture of 4-oxa-2,6-heptanediol, 2-(2-hydroxy-propoxy)-propan-1-ol, and 2-(2-hydroxy-1-methyl-ethoxy)-propan-1-ol), 1,3-butanediol, glycerin (propane-1,2,3-triol), and polyethylene glycol (having, for example, average molecular weight 200 to 400); ketones such as methyl isobutyl ketone; ethers such as 1,4-dioxane and diethylene glycol dimethyl ether (i.e. diglyme); halogenated hydrocarbons such as chlorobenzene; aromatic hydrocarbons such as toluene, benzene, xylene, and ethylbenzene; nitriles such as propionitrile; aprotic polar solvents such as DMF, NMP, N,N-dimethylacetamide, 1,3-dimethyl-2-imidazolidinone, and DMSO; and mixtures thereof.

Examples of the acid used in the reaction include sulfonic acids such as p-toluenesulfonic acid; carboxylic acids such as acetic acid and lactic acid; and sulfuric acid, phosphoric acid, and polyphosphoric acid.

Also, when the reaction mixture obtained in the Step (A) is mixed with water in the work-up of the Step (A), phosphoric acid is produced in the reaction system by the reaction of phosphorus oxychloride with water and may be used as the acid in the Step (B).

In the reaction, 0.1 to 5 moles of the acid are usually used per one mole of the compound (5).

When the acid is a liquid such as lactic acid and acetic acid in the reaction, the acid may be used as a solvent, and the amount thereof to be used is usually 1 to 5 parts by weight per one part by weight of the compound (5) .

The reaction temperature is usually within the range of 100 to 200°C. The reaction time is usually within the range of 0.1 to 48 hours.

When the reaction is completed, for example, the reaction mixture is mixed with water, and then extracted with organic solvent(s), and the obtained organic layer is concentrated; the reaction mixture is mixed with water, and the obtained solids are collected by filtration; or solids produced in the reaction mixture are collected by filtration, to isolate the compound (6). The isolated compound (6) may be further purified by recrystallization, chromatography, or the like.

Next, Step (C) is described.

The reaction of Step (C) is usually carried out in a solvent.

Examples of the solvent include water; ethers such as 1,4-dioxane, diethyl ether, THF, and tert-butyl methyl ether; halogenated hydrocarbons such as dichloromethane, chloroform, carbon tetrachloride, 1,2-dichloroethane, and chlorobenzene; aromatic hydrocarbons such as toluene, benzene, xylene, and ethylbenzene; esters such as ethyl acetate and butyl acetate; nitriles such as acetonitrile; aprotic polar solvents such as DMF, N,N-dimethylacetamide, NMP, 1,3-dimethyl-2-imidazolidinone, and DMSO; nitrogen-containing aromatic compounds such as pyridine and quinoline; and mixtures thereof.

Examples of the inorganic base include alkali metal hydrides such as sodium hydride and potassium hydride; alkali metal carbonates such as sodium carbonate and potassium carbonate; and alkali metal hydroxides such as potassium hydroxide and sodium hydroxide.

In the reaction, 1 to 2 moles of 1H-1,2,4-triazole are usually used, and 1 to 5 moles of the base are usually used, per one mole of the compound (1).

The reaction temperature is usually within the range of 0 to 120°C. The reaction time is usually within the range of 0.1 to 24 hours.

After the compound (1) is reacted with 1H-1,2,4-triazole, the obtained reaction mixture is mixed with an acid. The amount of the acid to be used is usually 2 to 8 moles per one mole of the compound (1).

When solids are produced by mixing the reaction mixture with the acid, the obtained solids are collected by filtration to isolate the compound (3). When a solid is not produced by mixing the reaction mixture with the acid, the mixture is extracted with organic solvent(s), and the obtained organic layer is, for example, concentrated to isolate the compound (3). The isolated compound (3) may be further purified by recrystallization, chromatography, or the like.

Examples of the acid to be used include hydrochloric acid and sulfuric acid.

The compound (3) may also be produced according to the following scheme.

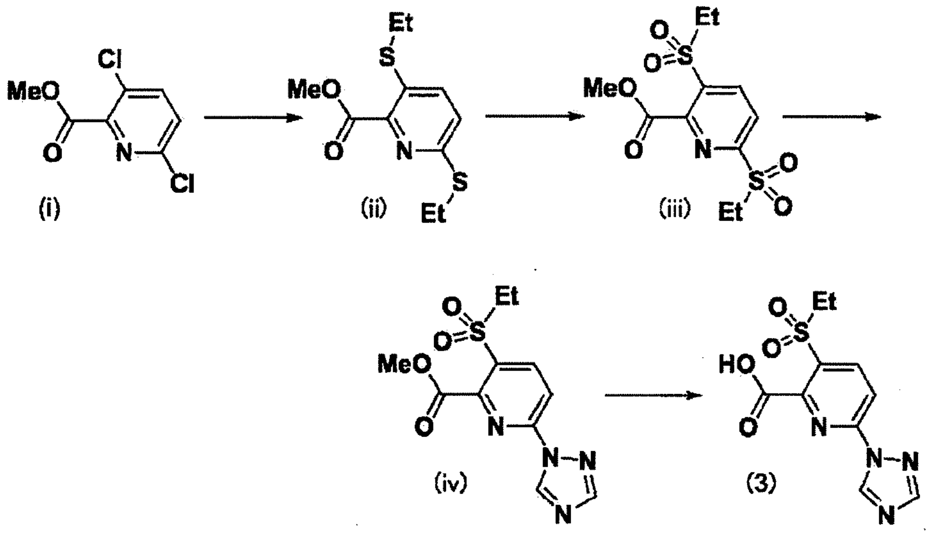

The compound represented by formula (ii) (hereinafter referred to as compound (ii)) can be produced by reacting the compound represented by formula (i) (hereinafter referred to as compound (i)) with ethanethiol in the presence of a base.

The reaction is usually carried out in a solvent.

Examples of the solvent include ethers such as THF, ethylene glycol dimethyl ether, tert-butyl methyl ether, and 1,4-dioxane; aromatic hydrocarbons such as toluene and xylene; nitriles such as acetonitrile; aprotic polar solvents such as DMF, NMP, and DMSO; water; and mixtures thereof.

Examples of the base include alkali metal carbonates such as sodium carbonate and potassium carbonate; alkali metal hydrides such as sodium hydride; and alkali metal hydroxides such as sodium hydroxide and potassium hydroxide.

In the reaction, 1 to 10 moles of ethanethiol are usually used, and 1 to 10 moles of the base are usually used, per one mole of the compound (i).

The reaction temperature is usually within the range of 0 to 100°C. The reaction time is usually within the range of 0.5 to 24 hours.

When the reaction is completed, the reaction mixture is subjected to work-up, for example, extracted with organic solvent(s), and the organic layer is dried or concentrated to isolate the compound (ii). The isolated compound (ii) may be further purified by chromatography, recrystallization, or the like.

The reaction mixture comprising the compound (ii) may be directly used in the next reaction.

The compound represented by formula (iii) (hereinafter referred to as compound (iii)) can be produced by reacting the compound (ii) with an oxidizing agent.

The reaction is usually carried out in a solvent.

Examples of the solvent include aliphatic halogenated hydrocarbons such as dichloromethane and chloroform; alcohols such as methanol and ethanol; acetic acid; water; and mixtures thereof.

Examples of the oxidizing agent include m-chloroperbenzoic acid and hydrogen peroxide solution.
The reaction may be carried out in the presence of a catalyst. Examples of the catalyst include sodium tungstate.
When a catalyst is used in the reaction, 2 to 5 moles of the oxidizing agent are usually used and 0.01 to 0.5 moles of the catalyst are usually used per one mole of the compound (ii), and preferably, 2 to 3 moles of the oxidizing agent are used per one mole of the compound (ii).
When a catalyst is not used in the reaction, 6 to 8 moles of the oxidizing agent are usually used per one mole of the compound (ii).
The reaction temperature is usually within the range of 0 to 120°C. The reaction time is usually within the range of 0.1 to 12 hours.
When the reaction is completed, the reaction mixture is subjected to work-up, for example, extracted with organic solvent(s), and if necessary, the organic layer is washed with an aqueous solution of a reducing agent (such as sodium sulfite and sodium thiosulfate) and then an aqueous solution of a base (such as sodium hydrogen carbonate), dried, and concentrated to isolate the compound (iii). The isolated compound (iii) may be further purified by chromatography, recrystallization, or the like.
The compound represented by formula (iv) (hereinafter referred to as compound (iv)) can be produced by reacting the compound (iii) with 1H-1,2,4-triazole in the presence of a base.
The reaction is usually carried out in a solvent.
Examples of the solvent include ethers such as 1,4-dioxane, diethyl ether, THF, and tert-butyl methyl ether; halogenated hydrocarbons such as dichloromethane, chloroform, carbon tetrachloride, 1,2-dichloroethane, and chlorobenzene; aromatic hydrocarbons such as toluene, benzene, and xylene; esters such as ethyl acetate and butyl acetate; nitriles such as acetonitrile; aprotic polar solvents such as DMF, NMP, 1,3-dimethyl-2-imidazolidinone, and DMSO; and mixtures thereof.
Examples of the base include alkali metal hydrides such as sodium hydride and potassium hydride; alkali metal carbonates such as sodium carbonate and potassium carbonate; and alkali metal hydroxides such as sodium hydroxide and potassium hydroxide.
the reaction, 1 to 2 moles of 1H-1,2,4-triazole are usually used, and 1 to 5 moles of the base are usually used, per one mole of the compound (iii).
The reaction temperature is usually within the range of 0 to 120°C. The reaction time is usually within the range of 0.1 to 24 hours.
When the reaction is completed, the reaction mixture is added to water, then extracted with organic solvent(s), and the obtained organic layer is concentrated to isolate the compound (iv). The isolated compound (iv) may be further purified by recrystallization, chromatography, or the like.
The reaction mixture comprising the compound (iv) may be directly used in the next reaction.
The compound (3) can be produced by hydrolyzing the compound (iv) under a basic condition.
Examples of the base include lithium hydroxide, sodium hydroxide, and potassium hydroxide.
The reaction temperature is usually within the range of 0 to 120°C. The reaction time is usually within the range of 0.1 to 24 hours.
When the reaction is completed, the reaction mixture is neutralized with an acid such as hydrochloric acid and sulfuric acid, and then, for example, (a) added to water and then extracted with organic solvent(s), and the obtained organic layer is concentrated; (b) added to water and the produced solids are collected by filtration; or (c) solids produced in the reaction mixture are collected by filtration, to isolate the compound (3). The isolated compound (3) may be further purified by recrystallization, chromatography, or the like.

## Examples

Hereinafter, the present invention is described more in detail by way of Examples, but the present invention is not limited to them. In the following Examples etc., "%" means "% by weight" unless otherwise specified.
In the following Examples, quantitative analysis was carried out by using high performance liquid chromatography unless otherwise specified. The analysis conditions are as follows.

High performance liquid chromatography analysis conditions 1

Internal standard material: isopropyl benzoate
Mobile phase: Solution A: 0.1% phosphoric acid aqueous solution, Solution B: acetonitrile

Solution A / Solution B = 65/35 (v/v)

Column: SUMIPAX (registered trademark) ODS Z-CLUE, Particle size 3 μm, 4.6 mm I.D. × 100 mm
UV measurement wavelength: 254 nm
Flow rate: 1.0 mL/min
Column oven: 40°C
Injection volume: 10 μL

High performance liquid chromatography analysis conditions 2

Internal standard material: biphenyl
Mobile phase: Solution A: 0.1% phosphoric acid aqueous solution, Solution B: acetonitrile
Gradient conditions: following Table 1
Column: SUMIPAX (registered trademark) ODS Z-CLUE, Particle size 3 μm, 3.0 mm I.D. × 100 mm
UV measurement wavelength: 254 nm
Flow rate: 1.0 mL/min
Column oven: 40°C
Injection volume: 10 μL

<Gradient conditions>

[Table 1]

| Time (min) | Solution B in mobile phase (%) |
|---|---|
| 0.00 | 10 |
| 30.00 | 90 |
| 35.00 | 90 |
| 35.01 | 10 |
| 40.00 | Stop |

High performance liquid chromatography analysis conditions 3

Mobile phase: Solution A: 0.05% trifluoroacetic acid aqueous solution, Solution B: acetonitrile
Gradient conditions: following Table 2
Column: SUMIPAX (registered trademark) ODS Z-CLUE, Particle size 3 μm, 4.6 mm I.D. × 100 mm
UV measurement wavelength: 260 nm
Flow rate: 1.0 mL/min
Column oven: 40°C
Injection volume: 10 μL

<Gradient conditions>

[Table 2]

| Time (min) | Solution B in mobile phase (%) |
|---|---|
| 0 | 5 |
| 5 | 5 |
| 45 | 80 |
| 45.01 | 5 |
| 60 | Stop |

Example 1

Under nitrogen atmosphere, the compound (4) (3.59 g, 18.8 mmol), NMP (5.11 g), and triethylamine (1.99 g) were mixed at room temperature. Each of the total amount of the resulting mixture and phosphorus oxychloride (2.74 g, 17.9 mmol) was simultaneously and separately added dropwise to a mixture of the compound (3) (5.12 g) and NMP (10.25 g) at 25°C over 7 hours, and each addition was simultaneously completed. Subsequently, the mixture was stirred at 25°C for 2 hours. Each drop rate of the compound (4) and phosphorus oxychloride was adjusted so that any one of them was not inappropriately excessively added, each addition of the compound (4) and phosphorus oxychloride was simultaneously started, roughly constant drop rate was kept, and each addition was simultaneously completed. The resulting mixture was analyzed by high performance liquid chromatography using isopropyl benzoate as internal standard material (High performance liquid chromatography analysis conditions 1) to confirm that the yield of the compound (5) was 93.2%.

To the resulting mixture was added water (36.1 g) at 25°C, and then the resulting solids were filtered. The filtered solids were washed with water (25.1 g), and then dried under reduced pressure to obtain the compound (5) as white solids (yield 82.4%).

$^1$H-NMR (DMSO-$D_6$) $\delta$: 10.46 (1H, br s), 9.84 (1H, s), 8.68 (1H, d, J = 8.6 Hz), 8.48 (1H, s), 8.38-8.37 (1H, br m), 8.25 (1H, d, J = 8.6 Hz), 7.93 (1H, d, J = 2.3 Hz), 6.66-6.64 (1H, br m), 3.72 (2H, q, J = 7.4 Hz), 2.95 (3H, d, J = 4.8 Hz), 1.22 (3H, t, J = 7.4 Hz)

Example 2

Under nitrogen atmosphere, the compound (4) (3.59 g, 18.8 mmol), NMP (5.11 g), and triethylamine (1.99 g) were mixed at room temperature. Each of the total amount of the resulting mixture and phosphorus oxychloride (2.98 g, 19.4 mmol) was simultaneously and separately added dropwise to a mixture of the compound (3) (5.12 g) and NMP (10.22 g) at 25°C over 20 hours, and each addition was simultaneously completed. Subsequently, the mixture was stirred at 25°C for 2 hours. Each drop rate of the compound (4) and phosphorus oxychloride was adjusted so that any one of them was not inappropriately excessively added, each addition of the compound (4) and phosphorus oxychloride was simultaneously started, roughly constant drop rate was kept, and each addition was simultaneously completed. The resulting mixture was analyzed by high performance liquid chromatography using isopropyl benzoate as internal standard material (High performance liquid chromatography analysis conditions 1) to confirm that the yield of the compound (5) was 96.2%.

To the resulting mixture was added water (36.1 g) at 25°C, and then the resulting solids were filtered. The solids were washed with water (25.1 g), and then dried under reduced pressure to obtain the compound (5) as white solids (yield 86.8%).

Example 3

Under nitrogen atmosphere, the compound (4) (29.9 g, 156 mmol), NMP (46.3 g), and triethylamine (16.8 g) were mixed at room temperature. Each of the total amount of the resulting mixture and phosphorus oxychloride (24.7 g, 161 mmol) was simultaneously and separately added dropwise to a mixture of the compound (3) (46.3 g, 164 mmol) and NMP (92.6 g) at 5°C over 5 hours, and each addition was simultaneously completed. Each drop rate of the compound (4) and phosphorus oxychloride was adjusted so that any one of them was not inappropriately excessively added, each addition of the compound (4) and phosphorus oxychloride was simultaneously started, roughly constant drop rate was kept, and each addition was simultaneously completed. Subsequently, the mixture was stirred at 5°C for 2 hours, then cooled to 0°C, and stirred for 13 hours. The resulting mixture was analyzed by high performance liquid chromatography using isopropyl benzoate as internal standard material (High performance liquid chromatography analysis conditions 1) to confirm that the yield of the compound (5) was 94.7%.

Each of the resulting mixture and a 48% potassium hydroxide aqueous solution was separately added dropwise to water (248.4 g) of 50°C over 3 hours. In the addition, drop rate was adjusted so that the pH of the slurry in the flask was kept between 6 and 7. The resulting slurry was cooled to 25°C over 2 hours, and then solids were filtered. The solids were washed twice with water (134.4 g), and then dried under reduced pressure to obtain the compound (5) as white solids (71.6 g, content 91.8%, yield 92.3%).

Example 4

Under nitrogen atmosphere, the compound (5) (0.20 g), concentrated sulfuric acid (45 mg), water (0.20 g), and DMF (0.40 g) were mixed at room temperature, and the mixture was warmed to 120°C and stirred for 9 hours. To the resulting mixture was added water, and the precipitated solids were filtered, washed with water, and then dried under

reduced pressure. The solids were analyzed by high performance liquid chromatography using biphenyl as internal standard material (High performance liquid chromatography analysis conditions 2) to confirm that the compound (6) was obtained as white solids (yield 86.4%). $^1$H-NMR ($CDCl_3$) $\delta$: 9.16 (1H, s), 8.81 (1H, br s), 8.72 (1H, d), 8.36 (1H, br s), 8.31 (1H, d), 8.21 (1H, s), 3.93 (3H, s), 3.82 (2H, q), 1.39 (3H, t)

Example 5

Under nitrogen atmosphere, the compound (4) (14.63 g), NMP (35.01 g), and triethylamine (8.53 g) were mixed at room temperature. Each of the total amount of the resulting mixture and phosphorus oxychloride (12.92 g) was simultaneously and separately added dropwise to a mixture of the compound (3) (20.01 g) and NMP (45.02 g) at 25°C over 7 hours, and each addition was simultaneously completed. Subsequently, the mixture was stirred at 25°C for 2 hours. Each drop rate of the compound (4) and phosphorus oxychloride was adjusted so that any one of them was not inappropriately excessively added, each addition of the compound (4) and phosphorus oxychloride was simultaneously started, roughly constant drop rate was kept, and each addition was simultaneously completed.

To the resulting mixture was added water (5.01 g) at 25°C, and the mixture was warmed to 40°C, stirred for 2 hours, then warmed to 130°C, and stirred for 21 hours. The resulting mixture was added dropwise to a 9.4% sodium hydrogen carbonate aqueous solution (260.12 g) kept at 60°C over 4 hours to precipitate solids. Subsequently, the resulting mixture was cooled to 25°C. The precipitated solids were filtered, the solids were washed twice with water (90 g), and then dried under reduced pressure to obtain the compound (6) as white solids (yield 86.0% based on compound (3)). The yield was measured by High performance liquid chromatography analysis conditions 1 using isopropyl benzoate as internal standard material.

Example 6

A mixture of the compound (5) (1.83 g), a 90% lactic acid aqueous solution (1.00 g), and propylene glycol (4.00 g) was warmed to 120°C, and stirred at 120°C for 12 hours. The resulting reaction mixture was cooled to 85°C, and water (5.00 g) was added thereto, and the mixture was stirred for 30 minutes. The mixture was cooled to room temperature, and the precipitated solids were filtered, washed with water (5.00 g), and dried under reduced pressure to obtain the compound (6) (1.78 g, content 92.6%, yield 93.8%: measured by High performance liquid chromatography analysis conditions 2 using biphenyl as internal standard material.).

Example 7

A mixture of a mixture of the compound (5) and water (water content 61.6%) (76.12 g), a 90% lactic acid aqueous solution (15.58 g), and propylene glycol (85.58 g) was heated in a bath at 135°C, dehydrated until the internal temperature became 120°C, and stirred for 11 hours. The resulting reaction mixture was cooled to 75°C, and water (142.46 g) was added thereto, and the mixture was stirred for 30 minutes. The mixture was cooled to 40°C, and the resulting solids were filtered, washed twice with water (143 g), and dried under reduced pressure to obtain the compound (6) (24.54 g, content 98.53%, yield 86.9%: measured by High performance liquid chromatography analysis conditions 1 using isopropyl benzoate as internal standard material.).

Powder X-ray diffraction chart of solid of the resulting compound (6) is shown in Figure 1.

The measurement conditions of X-ray diffraction are as follows.

Powder X-ray diffraction device: SmartLab (manufactured by Rigaku Corporation)
X-ray output: CuK$\alpha$, 45 kV, 200 mA
Sampling width: 0.02°
Scan range: 5° to 50°

Example 8

A mixture of the compound (5) (1.83 g) and a 90% lactic acid aqueous solution (4.00 g) was warmed to 120°C, and stirred at 120°C for 10 hours. The resulting reaction mixture was cooled to 85°C, and water (5.00 g) was added thereto, and the mixture was stirred for 30 minutes. The reaction mixture was cooled to room temperature, and the precipitated solids were filtered, washed with water (5.00 g), and dried under reduced pressure to obtain the compound (6) (1.80 g, content 87.8%, yield 89.5%: measured by High performance liquid chromatography analysis conditions 2 using biphenyl as internal standard material.).

Example 9

A mixture of the compound (5) (1.83 g), a 90% lactic acid aqueous solution (1.00 g), and xylene (4.00 g) was warmed to 150°C, and stirred at 150°C for 10 hours. The resulting reaction mixture was concentrated, and to the residues was added water (5.00 g), and the mixture was stirred for 30 minutes. The precipitated solids were filtered, washed with water (5.00 g), and dried under reduced pressure to obtain the compound (6) (1.68 g, content 89.3%, yield 85.2%: measured by High performance liquid chromatography analysis conditions 2 using biphenyl as internal standard material.).

Example 10

A mixture of the compound (5) (1.83 g), a 90% lactic acid aqueous solution (1.00 g), and methyl isobutyl ketone (4.00 g) was warmed to 120°C, and stirred at 120°C for 43 hours. The resulting reaction mixture was concentrated, and to the residues was added water (5.00 g), and the mixture was stirred for 30 minutes. The precipitated solids were filtered, washed with water (5.00 g), and dried under reduced pressure to obtain the compound (6) (1.65 g, content 84.6%, yield 79.5%: measured by High performance liquid chromatography analysis conditions 2 using biphenyl as internal standard material.).

Example 11

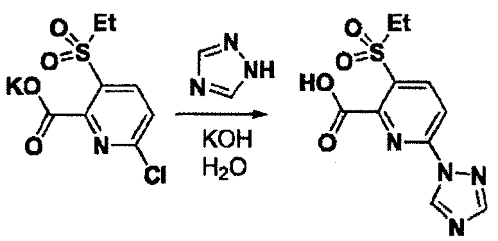


Under nitrogen atmosphere, a potassium 6-chloro-3-(ethanesulfonyl)-2-pyridinecarboxylate aqueous solution (content: 38.9%) (200.00 g), 1H-1,2,4-triazole (37.70 g), and potassium hydroxide (21.26 g) were mixed at room temperature, and the mixture was stirred at 85°C for 9 hours. The resulting mixture was cooled to 55°C, and then water (194.35 g) was added thereto, and then concentrated sulfuric acid (59.48 g) was added dropwise thereto over 6 hours. The mixture was cooled to 20°C, and the resulting solids were filtered, and washed with water (116.6 g). Subsequently, the solids were dried under reduced pressure to obtain the compound (3) as white solids (yield 96.1%). The yield was measured by absolute calibration curve method using High performance liquid chromatography analysis conditions 3.

Example 12

Under nitrogen atmosphere, a potassium 6-chloro-3-(ethanesulfonyl)-2-pyridinecarboxylate aqueous solution (content: 38.9%) (150.10 g) and 1H-1,2,4-triazole (28.27 g) were mixed at room temperature, and the mixture was warmed to 85°C. To the resulting mixture was added dropwise a 48% potassium hydroxide aqueous solution (33.26 g) at 85°C over 3.5 hours, and then the mixture was stirred at 85°C for 5 hours, and then cooled to 55°C. The resulting mixture was added dropwise to a mixture of water (145.8 g) and concentrated sulfuric acid (44.61 g) kept at 55°C over 4 hours. The resulting mixture was cooled to 20°C, and the resulting solids were filtered, and washed with water (87.5 g). Subsequently, the solids were dried under reduced pressure to obtain the compound (3) as white solids (yield 96.9%). The yield was measured by absolute calibration curve method using High performance liquid chromatography analysis conditions 3.

Reference example 1

Under nitrogen atmosphere, the compound (3) (5.12 g), the compound (4) (3.59 g), NMP (15.41 g), and triethylamine (2.01 g) were mixed at room temperature. To the mixture was added dropwise phosphorus oxychloride (3.02 g) at 25°C over 20 hours, and then the mixture was stirred at 25°C for 2 hours. The resulting mixture was analyzed by high performance liquid chromatography using isopropyl benzoate as internal standard material (High performance liquid chromatography analysis conditions 1) to confirm that the yield of the compound (5) was 78.3%.

To the resulting mixture was added water (25.1 g) at 25°C, and the precipitated solids were filtered, washed with water (25.3 g), and then dried under reduced pressure to obtain the compound (5) as white solids (yield 71.4%). The yield was measured by High performance liquid chromatography analysis conditions 1 using isopropyl benzoate as internal standard material.

Example 13

(1) To a mixture of methyl=3,6-dichloropyridine-2-carboxylate (3.0 g), ethanethiol (2.27 mL), and THF (29 mL) was added sodium hydride (1.28 g) under ice-cooling. The mixture was stirred at room temperature for 1 hour, and then to the mixture was added a saturated ammonium chloride aqueous solution, and then the mixture was extracted with ethyl acetate. The organic layer was dried over sodium sulfate, and then concentrated. The residues were subjected to silica gel column chromatography to obtain methyl=3,6-bis(ethylsulfanyl)pyridine-2-carboxylate (2.80 g).

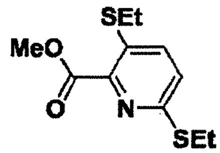

$^1$H-NMR ($CDCl_3$) δ: 7.53 (1H, d, J = 8.6 Hz), 7.22 (1H, d, J = 8.4 Hz), 3.97 (3H, s), 3.19 (2H, q, J = 7.3 Hz), 2.90 (2H, q, J = 7.4 Hz), 1.38 (3H, t, J = 7.4 Hz), 1.34 (3H, t, J = 7.4 Hz)

(2) To a mixture of methyl=3,6-bis(ethylsulfanyl)pyridine-2-carboxylate (2.80 g) and chloroform (36 mL) was added m-chloroperbenzoic acid (10.28 g) under ice-cooling. The mixture was stirred at room temperature for 8 hours, and then a saturated sodium thiosulfate aqueous solution and saturated sodium bicarbonate water were sequentially added thereto, and the mixture was extracted with ethyl acetate. The organic layer was dried over sodium sulfate, and then concentrated to obtain methyl=3,6-bis(ethanesulfonyl)pyridine-2-carboxylate (3.43 g).

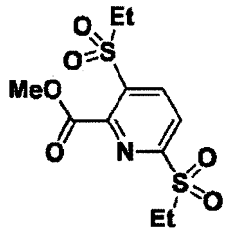

$^1$H-NMR ($CDCl_3$) δ: 8.63 (1H, d, J = 8.0 Hz), 8.36 (1H, d, J = 8.2 Hz), 4.05 (3H, s), 3.55 (2H, q, J = 7.4 Hz), 3.52 (2H, q, J = 7.4 Hz) 1.38 (3H, t, J = 7.4 Hz), 1.37 (3H, t, J = 7.4 Hz)

(3) To a mixture of methyl=3,6-bis-ethanesulfonyl-2-pyridinecarboxylate (1.0 g) and DMF (10 mL) were added 1H-1,2,4-triazole (237 mg) and potassium carbonate (473 mg) at room temperature. The mixture was stirred at room temperature for 5 hours, and then to the mixture was added a saturated ammonium chloride aqueous solution, and the mixture was extracted with ethyl acetate. The organic layer was dried over sodium sulfate, and then concentrated to obtain methyl=3-(ethanesulfonyl)-6-(1H-1,2,4-triazol-1-yl)pyridine-2-carboxylate (790 mg).

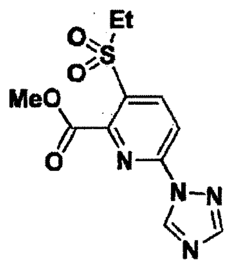

$^1$H-NMR ($CDCl_3$) δ: 9.24 (1H, s), 8.51 (1H, d, J = 8.5 Hz), 8.18-8.17 (2H, m), 4.07 (3H, s), 3.51 (2H, q, J = 7.5 Hz), 1.37 (3H, t, J = 7.4 Hz)

(4)

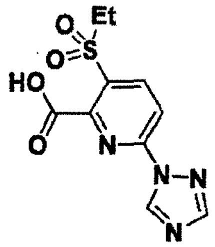

To a mixture of methyl=3-(ethanesulfonyl)-6-(1H-1,2,4-triazol-1-yl)pyridine-2-carboxylate (790 mg), water (6 mL), and THF (10 mL) was added lithium hydroxide (128 mg) at room temperature. The mixture was stirred at room temperature for 3 hours, and then 1N hydrochloric acid was added thereto, and then the mixture was extracted with ethyl acetate. The organic layer was dried over sodium sulfate, and then concentrated to obtain the compound (3) (473 mg).

$^{1}$H-NMR ($CDCl_3$) δ: 9.42 (1H, s), 8.56 (1H, d, J = 8.6 Hz), 8.23 (1H, s), 8.18 (1H, d, J = 8.6 Hz), 3.62 (2H, q, J = 7.5 Hz), 1.36 (3H, t, J = 7.5 Hz).

Reference example 2

(1)

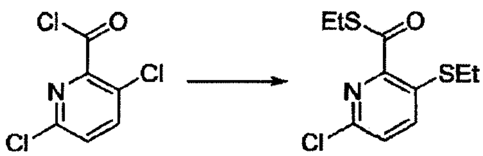

Under nitrogen atmosphere, to a mixture of tetrabutylammonium bromide (1.68 g), sodium hydroxide (4.60 g, purity 95%), water (10 g), and toluene (10 g) was added dropwise ethyl mercaptan (6.80 g) at room temperature over 10 minutes, and the mixture was stirred at the same temperature for 1 hour. To the reaction mixture was added dropwise a solution of 3,6-dichloropyridine-2-carboxylic acid chloride in toluene (20.00 g comprising 10.96 g of pure 3,6-dichloropyridine-2-carboxylic acid chloride) at 20°C over 30 minutes, and the mixture was stirred at the same temperature for 5 hours. The mixture was separated, and then the aqueous layer was removed, and the organic layer was washed with water (10 g). The organic layer was concentrated under reduced pressure to obtain S-ethyl 6-chloro-3-(ethylthio)pyridine-2-thiocarboxylate (13.59 g, content 85.82%). The content of S-ethyl 3-chloro-6-(ethylthio)pyridine-2-thiocarboxylate was 4.3%, and the content of S-ethyl 3,6-di(ethylthio)pyridine-2-thiocarboxylate was 1.8% (measured by absolute calibration curve method using High performance liquid chromatography analysis conditions 3).

$^{1}$H-NMR (DMSO-d6) δ: 1.22-1.29 (6H, m), 2.89-2.94 (2H, q), 2.99-3.05 (2H, q), 7.72-7.74 (1H, m), 7.98-8.00 (1H, m)

(2)

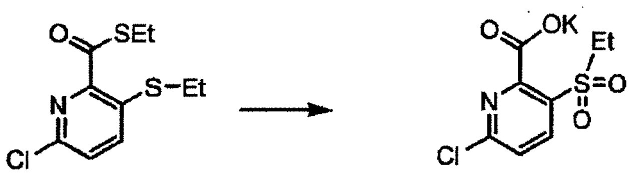

To a mixture of S-ethyl 6-chloro-3-(ethylthio)pyridine-2-thiocarboxylate (111.69 g, purity 89.53%), sodium tungstate dihydrate (2.52 g), ethylenediaminetetraacetic acid disodium salt dihydrate (2.84 g), 96% sulfuric acid (3.06 g), toluene (25.0 g), and water (10.0 g) was added dropwise a 60.0% hydrogen peroxide solution (125.6 g) at 50°C over 8 hours, and the mixture was stirred at the same temperature for 1 hour. Subsequently, a 22% sodium sulfite aqueous solution (33.1 g) was added thereto at room temperature, and a 48% sodium hydroxide aqueous solution was added thereto to adjust the pH of the reaction mixture to 0.6, and the mixture was extracted with methyl isobutyl ketone. To the resulting organic layer was added a potassium hydroxide aqueous solution until the pH of the aqueous layer became

5.5, and the mixture was stirred at room temperature for 30 minutes, and then separated to obtain an aqueous solution (260.4 g) comprising potassium 6-chloro-3-(ethylsulfonyl)pyridine-2-carboxylate (96.4 g).

$^1$H-NMR (DMSO-d6) δ: 1.05-1.09 (3H, t), 3.69-3.75 (2H, q), 7.51-7.53 (1H, d), 8.09-8.11 (1H, d)

Reference example 3

(1)

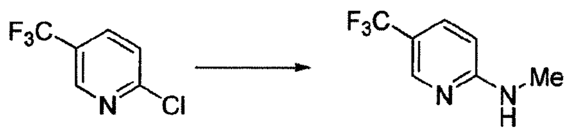

To a 1 L autoclave were added 2-chloro-5-trifluoromethylpyridine (168.2 g) and acetonitrile (50.5 g), and a 40% methyl amine aqueous solution (215.9 g) was pressed into the mixture over 6 hours at 85°C under sealed conditions, and the mixture was stirred at the same temperature for 8 hours. Subsequently, to the mixture was added acetonitrile (34.0 g), and added seed crystals of N-methyl-(5-trifluoromethyl-pyridin-2-yl)amine (0.17 g) at 53°C, and the mixture was incubated for 2 hours. The mixture was cooled to 30°C at the rate of 5°C/hour, and then water (336.0 g) was added dropwise thereto over 2 hours, and the mixture was cooled to 5°C at the rate of 10°C/hour, and stirred at the same temperature overnight. The precipitates were filtered, then washed with water (168.0 g), and then dried at 40°C under reduced pressure to obtain N-methyl-(5-trifluoromethyl-pyridin-2-yl)amine (159.8 g, content 99.5%, yield 97.4%).

(2)

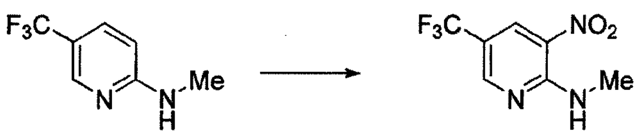

To a mixture of N-methyl-(5-trifluoromethyl-pyridin-2-yl)amine (135.3 g, content 99.5%), sulfolane (135.2 g), silica gel (4.1 g), and 98% sulfuric acid (338.3 g) was added dropwise 98% nitric acid (103.7 g) at 50°C over 12 hours, and then the mixture was stirred at the same temperature for 48 hours. The resulting reaction mixture was poured into water (270.6 g) at 45°C over 4 hours, and then a 48% sodium hydroxide aqueous solution (281.7 g) was added dropwise thereto at the same temperature over 6 hours. Xylene (257.1 g) was added thereto, and the mixture was stirred for 1 hour, then allowed to stand, and separated to remove the aqueous layer. Water (135.3 g) was added thereto, and then a 48% sodium hydroxide aqueous solution was added thereto until the pH of the aqueous layer became 10.3. The mixture was stirred for 45 minutes, then allowed to stand, separated, and the aqueous layer was removed to obtain a xylene solution of N-methyl-(3-nitro-5-trifluoromethyl-pyridin-2-yl)amine (407.1 g). The solution was analyzed using high performance liquid chromatography to confirm that the content of N-methyl-(3-nitro-5-trifluoromethyl-pyridin-2-yl)amine was 29.6% (yield 71.2%).

(3)

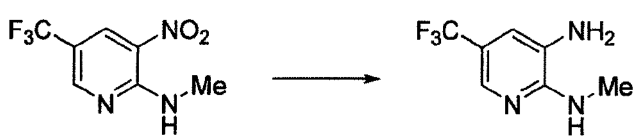

To a 1 L autoclave were added the xylene solution of N-methyl-(3-nitro-5-trifluoromethyl-pyridin-2-yl) amine (387.8 g) obtained in (2), xylene (95.2 g), and 3% platinum carbon (wetted with 50% water) (4.0 g), and the mixture was stirred at 40 to 45°C for 6 hours under hydrogen atmosphere of about 0.5 atm. The reaction mixture was filtered through Celite (registered trademark), and the residues were washed with xylene. The resulting filtrate and wash liquids were mixed, and the mixture was concentrated under reduced pressure until the content of N$^2$-methyl-5-trifluoromethylpyridine-2,3-diamine (compound (4)) became 40%. Subsequently, seed crystals of the compound (4) (0.11 g) were added at

52°C, and the mixture was incubated for 2 hours. Heptane (148.5 g) was added dropwise thereto at the same temperature over 3 hours, and the mixture was cooled to 5°C at the rate of 7°C/hour, and stirred at the same temperature overnight. The precipitates were filtered, then washed with heptane (80.3 g), and then dried at 40°C under reduced pressure to obtain the compound (4) (99.4 g, content 90.8%, yield 91.0%).

Industrial Applicability

The present invention provides a production intermediate of the compound (6) having control efficacies against pests and a method for producing the compound.

## Claims

**1.** A method for producing a compound represented by formula (5)

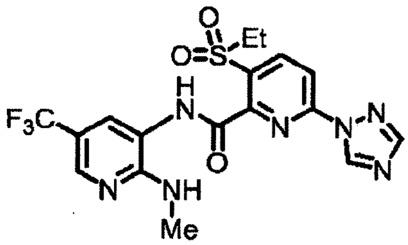

(5)

, the method comprising
Step (A): adding a compound represented by formula (4)

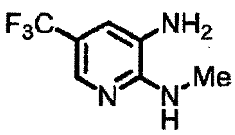

(4)

and phosphorus oxychloride simultaneously and separately to a compound represented by formula (3)

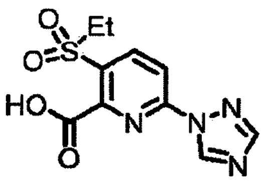

(3)

to produce the compound represented by formula (5).

**2.** A method for producing a compound represented by formula (6)

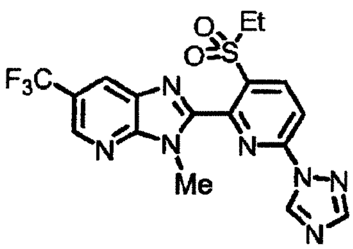

(6)

, the method comprising

Step (A) according to claim 1; and
Step (B) : subjecting said compound represented by formula (5) to intramolecular condensation in the presence of an acid to produce said compound represented by formula (6).

3. The method according to claim 1 or 2, the method comprising
Step (C): reacting a compound represented by formula (1)

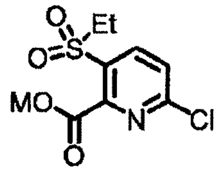

(1)

wherein M represents a sodium atom or a potassium atom with 1H-1,2,4-triazole in the presence of an inorganic base to obtain a reaction mixture, and then mixing the reaction mixture with an acid to produce the compound represented by formula (3).

4. A compound represented by formula (3):

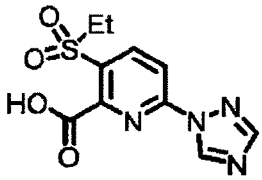

(3)

.

## Patentansprüche

1. Ein Verfahren zur Herstellung einer durch Formel (5) dargestellten Verbindung

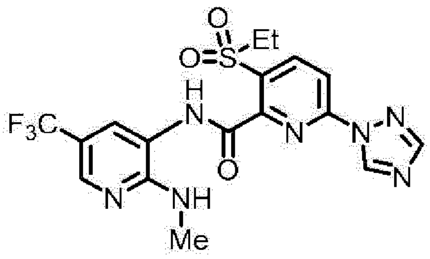

(5)

wobei das Verfahren umfasst
Schritt (A): Zugeben einer durch Formel (4) dargestellten Verbindung

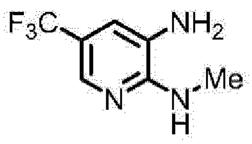

(4)

und Phosphoroxychlorid gleichzeitig und getrennt voneinander zu einer durch Formel (3) dargestellten Verbindung

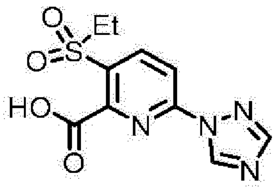

(3)

um die durch Formel (5) dargestellte Verbindung herzustellen.

**2.** Ein Verfahren zur Herstellung einer durch Formel (6) dargestellten Verbindung

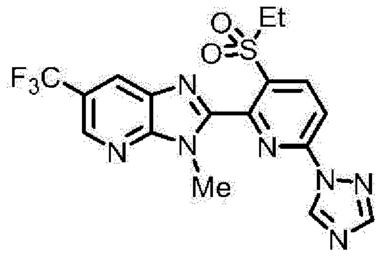

(6)

wobei das Verfahren umfasst

Schritt (A) gemäß Anspruch 1; und
Schritt (B): Unterziehen der durch Formel (5) dargestellten Verbindung einer intramolekularen Kondensation in Gegenwart einer Säure, um die durch Formel (6) dargestellte Verbindung herzustellen.

**3.** Das Verfahren gemäß Anspruch 1 oder 2, wobei das Verfahren umfasst
Schritt (C): Umsetzen einer durch Formel (1) dargestellten Verbindung

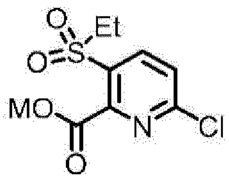

(1)

wobei M ein Natriumatom oder ein Kaliumatom darstellt
mit 1H-1,2,4-Triazol in Gegenwart einer anorganischen Base, um ein Reaktionsgemisch zu erhalten, und anschließendes Mischen des Reaktionsgemisches mit einer Säure, um die durch Formel (3) dargestellte Verbindung herzustellen.

**4.** Eine Verbindung, dargestellt durch Formel (3):

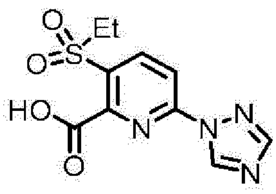

(3) .

## Revendications

**1.** Procédé pour produire un composé représenté par la formule (5)

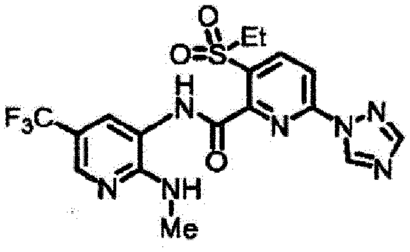

(5)

le procédé comprenant
étape (A) : addition d'un composé représenté par la formule (4)

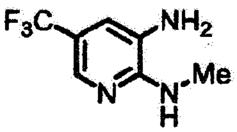

(4)

et d'oxychlorure de phosphore simultanément et séparément à un composé représenté par la formule (3)

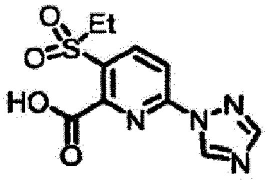

(3)

pour produire le composé représenté par la formule (5).

**2.** Procédé pour produire un composé représenté par la formule (6)

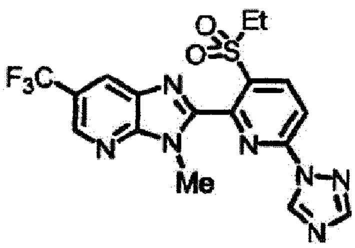

(6)

le procédé comprenant

étape (A) selon la revendication 1 ; et
étape (B) : soumission dudit composé représenté par la formule (5) à une condensation intramoléculaire en présence d'un acide pour produire ledit composé représenté par la formule (6).

**3.** Procédé selon la revendication 1 ou 2, le procédé comprenant
étape (C) : réaction d'un composé représenté par la formule (1)

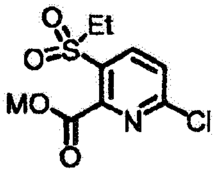

(1)

où M représente un atome de sodium ou un atome de potassium avec le 1H-1,2,4-triazole en présence d'une base inorganique pour obtenir un mélange réactionnel, puis mélange du mélange réactionnel avec un acide pour produire le composé représenté par la formule (3).

4. Composé représenté par la formule (3) :

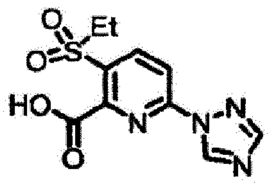

(3)

Figure 1

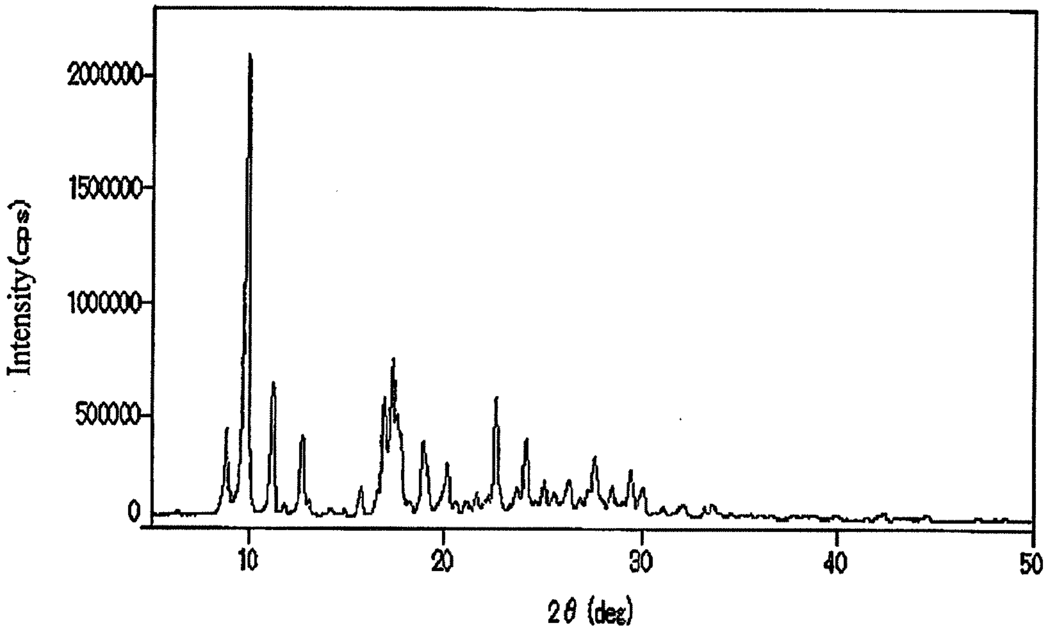
2000000
1500000
1000000
500000
0
Intensity (cps)
10
20
30
40
50
2θ (deg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013018928 A